# EUROPEAN PATENT SPECIFICATION

(11) **EP 2 482 899 B1**
(45) Date of publication and mention of the grant of the patent: **18.09.2013**
(21) Application number: 10765992.2
(22) Date of filing: 29.09.2010
(51) Int. Cl.: A61M 5/315

(54) **ASSEMBLY FOR A DRUG DELIVERY DEVICE**
ANORDNUNG FÜR EINE ARZNEIMITTELABGABEVORRICHTUNG
ASSEMBLAGE POUR DISPOSITIF D'ADMINISTRATION

(30) Priority: 30.09.2009 EP 09171761
(43) Date of publication of application: 08.08.2012
(73) Proprietor: Sanofi-Aventis Deutschland GmbH, 65929 Frankfurt (DE)
(72) Inventor: KOUYOUMJIAN, Garen, Warwickshire CV31 1HN (GB); VEASEY, Robert, Warwickshire CV32 7EP (GB); PLUMPTRE, David, Worcestershire WR9 7RQ (GB); JONES, Christopher, Gloucestershire GL20 7AT (GB); MACDONALD, Catherine, Anne, Warwickshire CV34 6JE (GB); JONES, Matthew, Warwickshire CV34 6DL (GB)
(74) Representative: KEIL & SCHAAFHAUSEN Patentanwälte
(86) International application number: PCT/EP2010/064422
(87) International publication number: WO 2011/039229

(56) References cited:
- EP-A1- 1 923 084
- WO-A1-2004/078241
- WO-A1-2006/024461

## Description

A drug delivery device is operable to deliver a drug from a cartridge e.g. via a cannula. A drive mechanism of the drug delivery device pushes a bung into the cartridge so that a predetermined dose is delivered.

The dose may be variable or fixed. The drug delivery device or parts of it may be disposable or reusable. When the drug delivery device is assembled or parts of the drug delivery device, e.g. the cartridge, are exchanged, the parts of the driving mechanism may be not positioned in a predetermined position with respect to the cartridge or a housing of the drug delivery device. If the parts are positioned in the predetermined position, this ensures that a predetermined dose is delivered when the drug delivery device is used the first time after assembling. After assembly there may be an internal gap between parts of the drug delivery device which have to contact each other to ensure the delivery of the correct amount of the dose. The gap may be located between parts of the drive mechanism or between a part of the drive mechanism and e.g. the cartridge. The gap is a consequence of tolerances associated with all the assembled parts.

In a conventional drug delivery device, a priming operation is performed to ensure that the parts of the driving mechanism are moved to their predetermined position with respect to the other parts.

Users who are unfamiliar with such drug delivery devices may fail to incorrectly prime their drug delivery device before dispensing the first dose. If this occurs, the user may inject the priming fluid or the correct volume of the drug may not be delivered in the first dose. This is because there is typically an internal gap between the end of the piston rod and the cartridge bung when a disposable drug delivery device is assembled or the cartridge of the reusable drug delivery device has been exchanged. This gap is a consequence of the tolerances associated with all the assembled parts and the requirement not to preload the bung axially in the assembled drug delivery device, because preloading would pressurise the drug in the cartridge.

It is an aim of the present invention to close this gap prior to the delivery of the device to the user, thereby eliminating the need for a priming operation prior to the dispense of the first dose by the user.

WO 2004/078241 A1 and WO 2006/024461 A1 disclose prior art devices.

For this aim an assembly for a drug delivery device is provided, the assembly comprising a first drive member for driving a piston rod. In a first state of the assembly the first drive member is moveable with respect to a second drive member. The piston rod is configured to be moved with respect to the first drive member when the first drive member is moved with respect to the second drive member. The assembly further comprises a coupling member which is configured to couple the first drive member with the second drive member in a second state of the assembly, so that the movement of the first drive member with respect to the second drive member is prevented, thereby forming a drive unit for a set and delivery action of the drug delivery device, which means that the first drive member and the second drive member do not move relative to each other during the set and delivery action.

This assembly can be adjusted during assembly, preferably during final assembly after fitting the cartridge, to bring the piston rod in contact with the cartridge bung. This removes tolerance gaps from the assembly and eliminates the need for a priming operation which is undertaken by the user prior to delivery the first dose of drug. The movement of the first and second drive member relative to each other enables adjustment of the drive mechanism before normal operation which means before the set and the delivery actions. The first and second drive members of the inventive assembly are connected during the set and delivery action so that they do not move relative to each other. In other words, during assembly, which is a setup phase, the first and second drive members can be adjusted and during the set and delivery action the first and second drive members effectively act as one.

A drug delivery device is suitable for delivery of one or more doses of drug contained in the cartridge. The doses may be fixed or variable. One embodiment of the drug delivery device is of the injector type suitable for injecting the drug to a patient or an animal. The drug delivery device is reusable or disposable. In one embodiment the drug delivery device is a pen-type drug delivery device. The pen-type drug delivery device has an elongated form which has a tubular or non-tubular shape. One embodiment is shaped mainly as ellipsoid. An alternative embodiment is mainly cubical.

The housing comprises exterior or interior housing parts which are designed to enable safe, correct, and comfortable handling of the drug delivery device. The housing may be a unitary or a multipart component. The housing comprises a cartridge which is configured to contain the drug. The cartridge may be fixed or engaged with outer or inner parts of the housing. A number of doses of the drug may be dispensed from the cartridge. An alternative embodiment of the cartridge is suitable for delivering a single dose.

A piston rod is moveable to push the bung of the cartridge along the inside wall of the cartridge during drug delivery, thereby delivering the drug.

The drive unit is suitable for driving the piston rod in the distal direction with respect to the housing during drug delivery. In one embodiment the drive unit transfers a movement which is impacted by the user during delivery operation to the piston rod. The drive unit comprises the first drive member and the second drive member which are connected during the set and delivery operation, so that first and second drive members do not move relative to each other. However, during final assembly the first drive member is moveable with respect to the second drive member, so that the piston rod is pushed towards to the bung by this movement.

The coupling member connects the first and the second drive member in the second state after final assembly so that they form the drive unit. In one embodiment the coupling member engages with at least one of the first and the second drive member in the first state such that the first drive member is moveable with respect to the second drive member.

In the first state of the assembly the first drive member may be rotationally moveable with respect to the second drive member. Rotational movement of the first drive member with respect to the second drive member results in axial movement of the piston rod, thereby adjusting the position of the piston rod. The first drive member and the piston rod are threadedly engaged, which enable the screwing of the piston rod towards the bung by rotating the first drive member.

In one embodiment the first drive member is at least partly positioned within the second drive member. The first drive member is an inner drive member and the second drive member is an outer drive member. This arrangement is compact and space-saving. The first and second drive member may be formed essentially tubular, so that the first drive member is rotationally moveable inside the second drive member.

In the first state of the assembly the coupling element may be moveable with respect to the second drive member, thereby moving the first drive member with respect to the second drive member. In other words, the first drive member is moved with respect to the second drive member when the coupling element is moved with respect to the second drive member. The coupling element is manipulated on the production line during assembly for rotating the first drive member.

In one embodiment the coupling member comprises a first clutch means and the first drive member comprises a second clutch means configured to be coupled with the first clutch means so that the first drive member rotates with respect to the second drive member when the coupling member rotates with respect to the second drive member. The first and second clutch means form a clutch connection suitable for transmitting rotation. In other words, the first drive member rotates with respect to the second drive member when the coupling member is rotated with respect to the second drive member in the first state. The first drive member and the coupling element are coupled in such a way that they spin at the same speed.

In one embodiment one of the second drive member and the coupling member is configured to engage with the other one of the second drive member and the coupling member in the first state so that the coupling member is rotationally moveable with respect to the second drive member in the first state of the assembly. One of the second drive member and the coupling member is configured to engage with the other one of the second drive member and the coupling member in the second state so that the coupling member is rotationally fixed with respect to the second drive member in the second state of the assembly. For engagement in the first state an arresting means and a detent means may be provided, the arresting means being formed as e.g. circumferential trench which engages with a detent spline, thereby enabling a rotational movement of the coupling member with respect to the second drive member.

In the second state, engagement means may prevent rotational movement in second state of the assembly. One of the second drive member and the coupling member may comprise a first engagement means configured to engage with a second engagement means which is comprised by the other one of the second drive member and the coupling member so that the movement of the first drive member with respect to the second drive member is prevented in the second state of the assembly. The engagement means can be connected so that rotational movement of the second drive member with respect to the coupling element is prevented. The engagement means may form a snapping connection or a clamped joint. The coupling member is in clutched connection with the first drive member so that the first and second drive members do not move with respect to each other.

When the coupling member is moved preferably axially with respect to the second drive member, the state of the assembly changes from the first state to the second state. Changing the states may be performed by a translational movement of the coupling member. In one embodiment the state of the assembly changes when the coupling element is axially moved with respect to the second drive member from a first detent position to a second detent position after adjustment of the position of the piston rod. Preferably, the movement of the coupling member relative to the drive member during the change of state differs from the movement of the components relative to one another during adjustment i.e. in one embodiments the adjustment is rotational while the state change is axial. Preferably, once the state is changed during assembly, it cannot be reversed, which means the user cannot pull the coupling member back out and interfere with the components.

One embodiment of the assembly described above may be used in a body element of a drug delivery device. In one embodiment a body element of a drug delivery device is configured to be coupled with a cartridge which comprises a bung. The assembly is positioned at least partly inside a housing of the body element, wherein the piston rod is configured to move towards the bung when the first drive member is moved with respect to the second drive member.

The body element is a part of the drug delivery device, the body element comprising the housing and the drive mechanism. The body element can be coupled to the cartridge or a cartridge holder. Alternatively the body element is integrally formed with the cartridge holder.

In one embodiment the piston rod is configured to move towards the bung and to abut the bung. Thereby, the piston rod is positioned on the bung before first use of the drug delivery device. The first dose can be used for injection after adjusting the position of the piston rod as described above.

In one embodiment the housing comprises a stop member configured to stop a distal movement of at least one of the first and second drive members with respect to the housing in the first state of the assembly. The drive members may be displaced in the distal direction with respect to the housing until it engages with the stop member. This step ensures that all mechanism tolerances are correctly taken up. The stop member may be formed as part of the housing extruding in the inside of the housing.

In one embodiment the drive unit is suitable for moving the piston rod in the distal direction with respect to the housing, thereby moving the bung in the distal direction with respect to the cartridge during the delivery action. The drive unit is formed by the first and second drive members which are mounted to each other in the second state.

Preferably, the coupling element serves as a button for initiating a set and delivery action of the drive mechanism. The button element is moveable by the user to initiate the delivery action, e.g. by pushing the button with respect to the housing during the delivery action. In one embodiment the button is also suitable for setting the dose, e.g. by rotating the button with respect to the housing. This twist-push action is used for setting and delivery in the second state of the assembly. In the first state the button is moved to adjust the mechanism. However, adjustment is not limited to a twist-push button but could be applied to other embodiments that may include a threaded drive member or button.

Furthermore a method for assembling a drug delivery device is provided. The drug delivery device comprises a body element having a drive mechanism, the body element being coupled with a cartridge having a bung. The method comprises the following steps. A first drive member is moved with respect to a second drive member, thereby moving a piston rod towards the bung. The first drive member is then coupled with the second drive member so that the movement of the first drive member with respect to the second drive member is prevented during a set and delivery action.

The term "drug", as used herein, preferably means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exedin-3 or exedin-4 or an analogue or derivative of exedin-3 or exedin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(02)25, Asp28] Exendin-4(1-39),
   des Pro36 [Trp(O2)25, IsoAsp28] Exendin-4(1-39),
   des Pro36 [Met(O)14 Trp(02)25, Asp28] Exendin-4(1-39),
   des Pro36 [Met(O)14 Trp(02)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(02)25, Asp28] Exendin-4(1-39),
   des Pro36 [Trp(02)25, IsoAsp28] Exendin-4(1-39),
   des Pro36 [Met(O)14 Trp(02)25, Asp28] Exendin-4(1-39),
   des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exedin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Other features will become apparent from the following detailed description when considered in conjunction with the accompanying drawings.

The scope of the invention is defined by the content of the claims. The invention is not limited to specific embodiments but comprises any combination of elements of different embodiments. Moreover, the invention comprises any combination of claims and any combination of features disclosed by the claims.
Figure 1 shows a three-dimensional cutaway view of a body element of an embodiment of a drug delivery device.
Figure 2 shows a cross-sectional view of the proximal part of a drug delivery device before final assembly.
Figure 3 shows a cross-sectional view of the proximal part of the drug delivery device after final assembly.
Figure 4 shows a three-dimensional cutaway view of a further embodiment of a drug delivery device.
Figure 5 shows a three-dimensional cutaway view of details of the further embodiment of the drug delivery device.

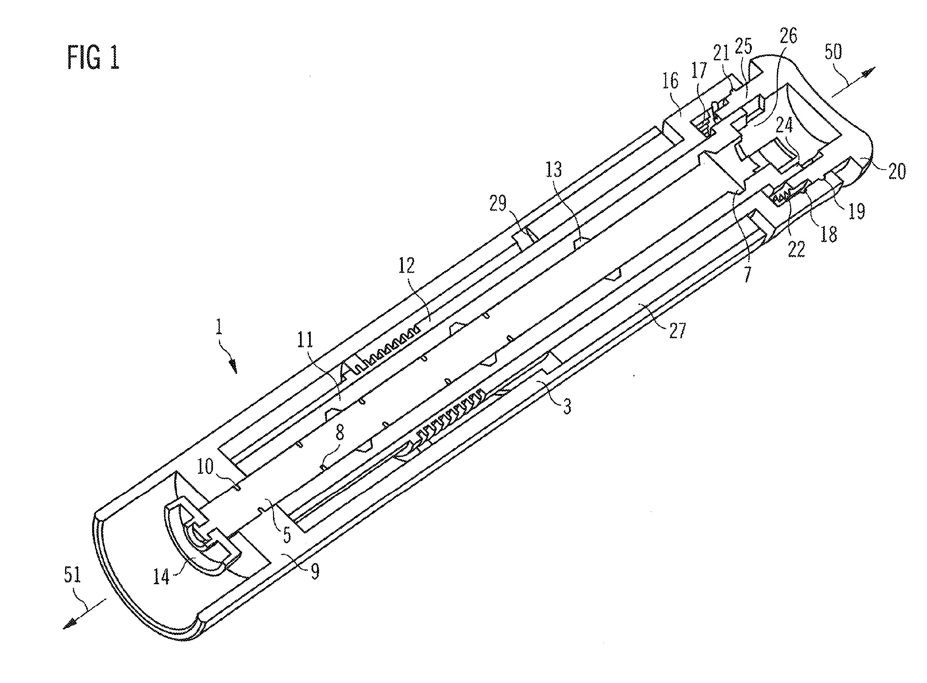

Figure 1 shows a three-dimensional cutaway view of a body element 1 of a drug delivery device, the body element 1 being the proximal part of the drug delivery device in which a drive mechanism for dispensing drug is located. The body element 1 comprises an adjustment mechanism which is suitable for adjusting the drive mechanism before first use of the drug delivery device.

The drug delivery device is designed as a pen-type drug delivery device. The pen-type drug delivery device has an elongated form which has a tubular or non-tubular shape.

One embodiment is shaped mainly as ellipsoid. An alternative embodiment is mainly cubical.

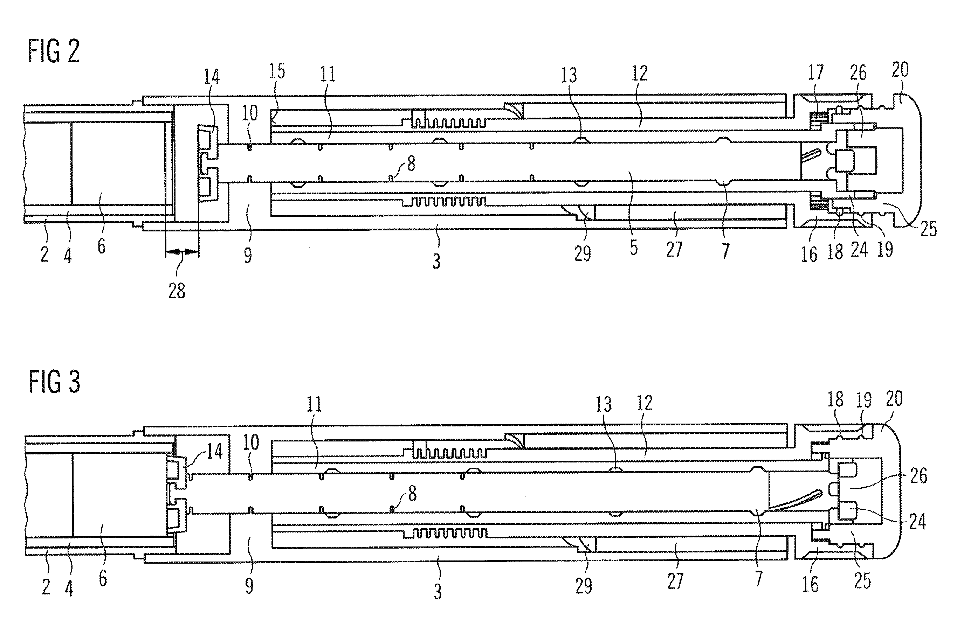

The drug delivery device comprises a housing having two main parts, a proximal housing 3 and a distal housing 2 (the latter is not shown in figure 1 but it is partly shown in figures 2 and 3). The distal end of the device is indicated by arrow 51, which refers to that end of the drug delivery device which is closest to a dispensing end of the drug delivery device. The proximal end of the device is indicated by arrow 50 referring to that end of the device which is furthest away from the dispensing end of the device. In this embodiment the proximal housing 3 is the housing of the body element 1.

One embodiment of the drug delivery device is disposable. An alternative embodiment is reusable, which means that at least a cartridge 4 (not shown in figure 1) which contains a drug is replaceable. Replacement of the cartridge may be performed by detaching the distal housing 2 (not shown in figure 1) or part thereof from the proximal housing 3, replacing the cartridge 4 and attaching the distal housing 2 to the proximal housing 3. In a further embodiment the distal housing 3 and the cartridge 4 are replaceable. In an alternative embodiment (not shown) the distal and proximal housings 2, 3 are formed in one piece. The cartridge 4 may be replaced through an opening located at the distal end of the housing. The drug delivery device may be designed for delivering fixed or variable doses.

The distal housing 2 serves as a cartridge holder. Within the distal housing 2 a drug cartridge 4 (not shown in figure 1) is located, which contains a number of doses of a liquid drug for injection. The cartridge 2 has a bung 6 (not shown in figure 1) located at the proximal end of the cartridge 4. The bung 6 is moveable along the inner side wall of the cartridge 4. An injection may be performed by means of a needle attached to the distal end of the cartridge 4. During use the distal housing 2, which serves as cartridge holder, is permanently attached to the proximal housing 3 of the drug delivery device.

The proximal housing 3 comprises a nut member 9 which is located at or near the distal end of the proximal housing 3. The nut member 9 is not moveable with respect to the proximal housing 3 and may be integrally formed with the proximal housing 3. In an alternative embodiment the nut member 9 is mounted between the distal and proximal housings 2, 3. The nut member 9 has a first thread 10 which is designed as internal thread.

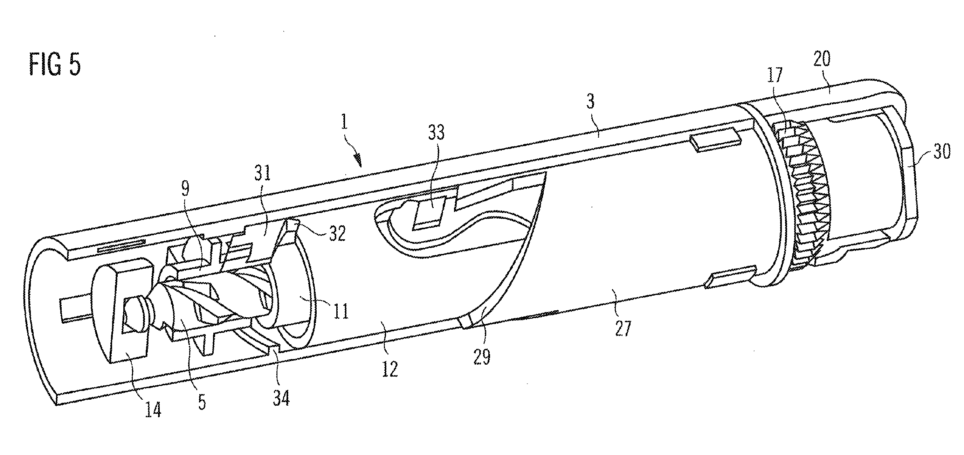

A body insert 27 having a mainly tubular shape is mounted inside the housing 1. The body insert 27 is not moveable with respect to the proximal housing 3. The distal edge of the body insert 27 serves as helical ramp 29. The pitch of the ramp 29 is identical to the pitch of the first thread 10. (The ramp 29 is clearly shown in figure 5.)

A drive mechanism is located substantially within the body element 1 of the drug delivery device. The drive mechanism comprises a lead screw 5 which serves as piston rod suitable for pushing the bung 6 along the inside wall of the cartridge 4 during the delivery action.

The lead screw 5 lies on a main axis of the drug delivery device and is initially located within the proximal housing 3. The lead screw 5 is essentially cylindrical in shape with a second thread 7 and a third thread 8 formed on the surface of the lead screw 5. The second and third threads 7, 8 may be positioned opposite hand. The second thread 7 is an external thread formed as a helical structure located at the proximal part of the lead screw 5. In this embodiment the helical structure comprises helical splines located on the outside of the lead screw 5. The third thread 8 is an external thread formed as a helical structure located at the distal part of the lead screw 5. In this embodiment the helical structure comprises helical trenches formed in the outside of the lead screw 5. In this embodiment the third thread 8 extends from the distal end of the lead screw 5 and the second thread 7 extends from the proximal end of lead screw. In one embodiment, the ranges along which the second and third threads 7, 8 extend overlap. In an alternative embodiment, the ranges along which the second and third threads 7, 8 extend do not overlap.

The third thread 8 is in threaded engagement with the first thread 10 of the nut member 9. Thus, the lead screw 5 moves axially with respect to the nut member 9, and the body element 1, when the lead screw 5 rotates with respect to the proximal housing 3.

A bearing 14 is connected to the distal end of the lead screw 5 by means of a journal that allows relative rotation between the bearing 14 and the lead screw 5, but no axial movement. In use, the bearing 14 is disposed to abut the proximal face of the cartridge bung 6 (not shown in figure 1). The bearing 14 prevents the transmission of torque to the bung 4 by the lead screw 5 during the delivery action.

The drive mechanism further comprises a drive unit which comprises a first drive member designed as an inner drive sleeve 11 and a second drive member designed as outer drive sleeve 12. The inner drive sleeve 11 lies on the main axis of the drug delivery device and is essentially tubular in shape. A fourth helical thread 13 is formed on the inner surface of the inner drive sleeve 11. The fourth helical thread 13 engages with the second thread 7 of the lead screw 5.

The outer drive sleeve 12 lies on the main axis of the drug delivery device and is essentially tubular in shape with a radially enlarged externally protruding barrel 16 at its proximal end. The outer drive sleeve 12 engages in a drive path formed between the proximal housing 3 and the body insert 27. A series of splines 17 are arranged radially around the internal cylindrical face at the barrel 16 of the outer drive sleeve.

A first and second arresting means 18, 19 are located proximally with respect to the splines 17. The first arresting means 18 is formed as a circumferential trench in the inner surface of the barrel 16. The second arresting means 19 is formed as a circumferential trench in the inner surface of the barrel 16, the second arresting means 19 being positioned proximally relative to the first arresting means 18.

A coupling lug (not shown in figure 1) is located on the outside of the outer drive sleeve 12. During the set action the outer drive sleeve 12 is helically moveable so that the coupling lug slides along the helical ramp 29.

A button 20 is located at the proximal end of the drug delivery device. The button 20 serves as a coupling element suitable for coupling the outer and the inner drive sleeves 12, 11. The button 20 comprises a proximal face suitable for being pressed by the user during the delivery action and a proximal part 25 which may have an essential tubular shape. The button 20 is mounted in the barrel 16 of the outer drive sleeve 12. The button 20 has a detent means which can engage with the first and second arresting means 18, 19. The detent means comprises a circumferential detent spline 21 which is located on the outer surface of the proximal part 25 of the button. In a first state the button 20 is arrested in a first detent position, as shown in figure 1, wherein the detent spline 21 engages with the second arresting means 19. In a second state the button 20 is arrested in a second detent position, which is axially displaced in the distal direction with respect to first detent position, wherein the detent spline 21 engages with the first arresting means 18. The arresting means and the detent means may be designed in another way, which is suitable for arresting the button 20 in the first and the second detent position. The detent means may comprise two circumferential splines (as shown in figures 2 and 3).

The button 20 has a set of splines 22 which are arranged radially around the external cylindrical surface of the proximal part 25 of the button. If the button 20 is positioned in the second detent position, the splines 22 of the button 20 and the splines 17 of the barrel 16 of the outer drive sleeve engage. If the button 20 is positioned in the first detent position, the splines 22 of the button and the splines 17 of the barrel do not engage.

The proximal part 25 of the button is in clutched engagement with the proximal part of the inner drive sleeve 11. At its proximal end the inner drive sleeve 12 has square profile clutch teeth 24 that are in engagement with matching clutch teeth 26 on the proximal part 25 of the button. This permits relative axial movement between the button 20 and the inner drive sleeve 11 but not relative rotational movement. In other words, the button 20 is axially moveable with respect to the inner drive sleeve 11, wherein the clutched engagement is not disconnected during the movement.

The button 20 is initially mounted in the first detent position, as shown in figure 1, such that the opposing sets of splines 17, 22 do not engage. When mounted in this position, relative rotation between the button 20 and the outer drive sleeve 12 is permitted. In addition, the button 20 is in clutched engagement with the inner drive sleeve 11 such that relative rotation is not permitted.

The inner and outer drive sleeve 11, 12 are positioned in the proximal housing 3. The nut member 9 serves as stop means which prevents distal movement of the inner and outer drive sleeves 11, 12.

The body element 1 shown in figure 1 is mounted during assembly to the distal part (not shown) of the drug delivery device which contains the cartridge. There is typically an intentional gap 28 (not shown in figure 1) between the end of the lead screw 5 and the cartridge bung 6 (not shown in figure 1). This gap 28 is a consequence of the tolerances associated with all the assembled parts.

Adjustment of the drive mechanism during final assembly to remove axial tolerances between the bearing 14 and the cartridge bung 6 will now be described with reference to figures 2 and 3.

Figure 2 shows a cross-sectional view of the drug delivery device before final assembly. Figure 3 shows a cross-sectional view of the drug delivery device after final assembly. The reference numerals are related to the same features as in figure 1.

When the drug delivery device sub-assembly is delivered for final assembly, which means insertion of the cartridge 4 and closure of the housing components 2, 3 the body element 1 is supplied with the button 20, the lead screw 5 and the drive mechanism in the configuration shown in figure 2. The button 20 is positioned in the first detent position.

During final assembly the cartridge 4 is first placed into the distal part 2 of the drug delivery device which serves as cartridge holder and the distal part 2 is then connected, e.g. irreversibly clipped, to the body element 1 of the drug delivery device. There is a gap 28 between the bearing 14 and the bung 6 of the cartridge before final assembly. The drive mechanism is then adjusted such that the gap 28 between the bearing 14 and the bung 6 is removed. This can be done using automated equipment.

Before final assembly the button 20 is positioned in the first detent state, as shown in Figure 2. The outer drive sleeve 12 is first displaced in the distal direction with respect to the housing 1 until the outer drive sleeve 12 engages its stop face 15 on the housing 1, which is the proximal face of the nut member 9. This step is important to ensure that all mechanism tolerances are correctly taken up. The outer drive sleeve 12 may be moved in the distal direction by gripping the barrel 16 of the outer drive sleeve and pushing it in the distal direction with respect to the proximal housing 3. Alternatively the outer drive sleeve 12 is moved in the distal direction by slightly pushing the button 20 in the distal direction so that the outer drive sleeve 12 is forced towards the stop face 15 but the button 20 remains in the first detent position.

The button 20 is then rotated relative to the outer drive sleeve 12. This action rotates the inner drive sleeve 11, which is in clutched connection with the button 20, with respect to the outer drive sleeve 12 and thereby advances the lead screw 5 through the first thread 10 of the nut member 9 until the bearing 14 contacts the bung 6. This can be detected e.g. by a strain gauge within the gripper head of an adjustment tool (not shown) which is used for rotating the button 20. The adjustment tool is suitable for measuring torque feedback which, on reaching a pre-determined level, signifies suitable contact between bearing 14 and bung 6. An alternative embodiment of the adjustment tool (not shown) measures the position of the bearing 14.

After abutting the bearing 14 against the bung 6, the button 20 is then pushed distally into the second detent position, as shown in figure 3. This action engages the two sets of splines 17, 22 between the button 20 and the outer drive sleeve 12, thus locking them to prevent rotation. As the inner drive sleeve 11 is already in clutched engagement with the button 20 this engagement also effectively locks the inner drive sleeve 12 rotationally to the outer drive sleeve 13. Thus the inner drive sleeve 11 and the outer drive sleeve 12 form a drive unit which is used during the set and delivery action in the normal operation mode of the drug delivery device. The drive unit 11, 12 is suitable for driving the lead screw 5 during the delivery action.

The adjustment of the drive mechanism may be performed by the manufacturer. If the manufacturer adjusts the drug delivery device, the user does not have to prime the device and so will not accidentally inject prime fluid. However, the button 20 which is positioned in the second detent position clearly indicates that the drug delivery device is ready to use. A priming step, e. g. delivering a primary dose which is not injected, is not necessary.

After final adjustment the drug delivery device can be used for the set and delivery action.

During the set action the barrel 16 or the button 20 is gripped by the user and rotationally moved with respect to the proximal housing 3 so that the coupling lug (not shown) slides along the helical ramp 29, which causes the button 20 and the proximal end of the drive unit which is formed by inner and outer drive sleeve 11, 12 to extend axially out of the proximal housing 3. The pitch of this helical ramp 29 is identical to the pitch of the helical interface between the first thread 10 and the lead screw 5, meaning that there is no movement of the lead screw 5 as the drive unit 11, 12 rotates over it.

Once the drug delivery device has been set, the user presses the button 20 axially in the distal direction with respect to the proximal housing 3 in order to dispense the dose. The threaded engagement between the inner drive sleeve 11 and the lead screw 5 causes the lead screw 5 to rotate and drive axially through its threaded engagement 10, 8 with the nut member 9, pushing in the distal direction on the bung 6 and thus dispensing the drug.

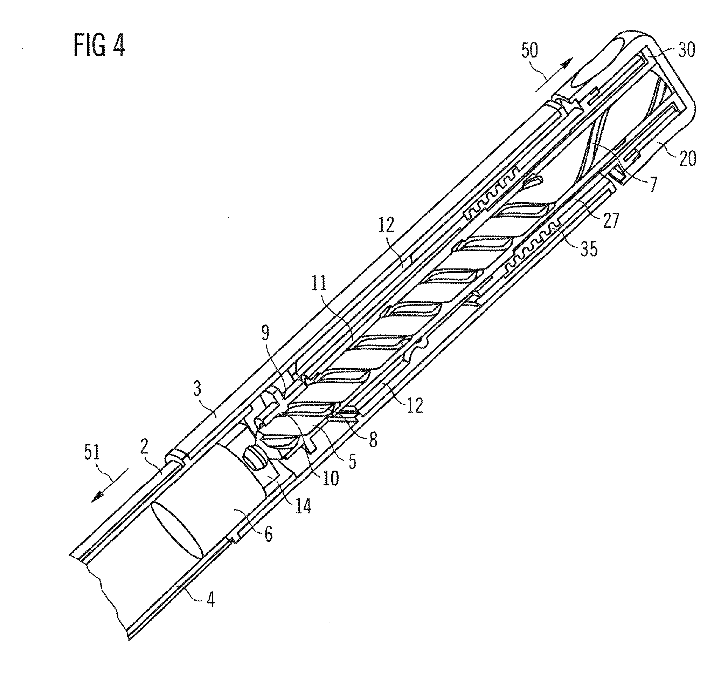

Figure 4 shows a three-dimensional cut away view of the proximal part of a further embodiment of the drug delivery device which comprises a further embodiment of a drive mechanism. The assembly and operation of this embodiment is similar to that described above. The reference numerals indicate features with the same or similar function. However, the internal components have been laid out differently in order to reduce complexity and to provide detail on user operation.

The button 20 is formed as a cap suitable to cover the proximal end of the outer drive sleeve 12. The button 20 is moveable from a first detent position to a second detent position, which is shown in figure 4. The arresting and detent means may be formed as described above.

In this embodiment the lead screw 5 is still driven in the distal direction by rotation of the button 20 which rotates the inner drive sleeve 11 independently of the outer drive sleeve 12, as described above. The proximal end 30 of the inner drive sleeve 11 extrudes from the outer drive sleeve 12 and is essential angled (clearly shown in figure 5), the proximal end 30 of the inner drive sleeve being positioned in a cavity of the button 20 which matches with the proximal end 30 of the inner drive sleeve, so that a rotational movement of the button 20 is transferred to the inner drive sleeve 11.

Subsequently the button 20 is pressed forward into splined engagement between the drive sleeves, locking the inner and outer drive sleeves 11, 12 together in rotation and preventing further rotation of the button 20 relative to the drive sleeves 11, 12. Splines (not explicitly shown in figure 4) located on the inside surface of the button 2 and splines 17 (not explicitly shown in figure 4, but shown in figure 5) located on the outside of the outer drive sleeve 12 engage when the button 2 is moved to the second detent position.

During normal operation the inner and outer drive sleeves 11, 12 are connected forming a drive unit for the set and delivery action. The user rotates the button 20 to set the drug delivery device. The rotation of the button 20 causes the outer drive sleeve 12 to rotate, acting at its distal end on a helical ramp 29 formed by the body insert 27 inside the proximal housing 3. As the button 20 is rotated, the reaction of the outer drive sleeve 12 on this ramp 29 causes the button 20 to extend axially out of the proximal housing 3. The pitch of this ramp 27 is identical to the pitch of the helical interface between the inner drive sleeve 11 and the lead screw 5, meaning that there is no movement of the lead screw 5 as the drive sleeves 11, 12 rotates over it.

Figure 5 shows a three-dimensional cut-out detailed view of the body element 1 of the drug delivery device. This embodiment of the drug delivery device comprises the outer drive sleeve 12 which has a distal spring means 31 with a cut-out 32 and a proximal spring means 33. The proximal housing 3 comprises a helical structure 34 forming a ramp along which the distal edge of the outer drive sleeve 12 can slide along. The function of these features are as follows.

At the end of the setting action, the distal spring means 30 located at the distal end of the outer drive sleeve 12 ride over radial ramps formed by helical structure 34 inside the proximal housing 3. This action provides the user with feedback that a dose has been set and the shape of the ramp ensures that the outer drive sleeve 12 cannot be "back-rotated", effectively un-setting the dose. During the setting action, the button 20 cannot be pulled purely axially by the user due to interference between the proximal spring means 33 located on the outer drive sleeve 12 and the distal edge 29 of the body insert 27. This interface is also helical, encouraging rotation of the outer drive sleeve 12 even when under a purely axial load in the proximal direction.

Once the drug delivery device has been set, the user presses the button 20 axially in the distal direction in order to dispense a dose. The threaded engagement between the inner drive sleeve 11 and the lead screw 5 causes the lead screw 5 to rotate and drive axially through its threaded engagement with the nut member 9, pushing in the distal direction on the bung 6 (not shown in figure 5) and thus dispensing drug.

At the end of axial travel, the proximal spring means 33 located on the outer drive sleeve 12 passes under the ramp on the body insert 27, giving the user feedback that the dose has been fully dispensed and to provide a non-return feature such that the user cannot pull the button 20 axially back up the dispense path it has just traveled and forcing any axial motion to set the next dose. Cut-outs 32 on the distal spring means 31 of the outer drive sleeve 12 allow the distal end of the outer drive sleeve 12 to flex axially and produce an axial spring that acts to back-off the outer drive sleeve 12, and hence the lead screw 5, from the proximal face of the bung 6 once axial load has been removed from the button 20.

In addition, the embodiment may comprise a last dose nut 35 (shown in figure 4) that is splined to the body insert 27 and threaded to the outer drive sleeve 12. As the device is set, the motion of the outer drive sleeve 12 relative to the body insert 27 causes the last dose nut to travel progressively down its thread on the outer drive sleeve 12. Once the last dose has been set on the device, the last dose nut 35 reaches the end of its thread, prohibiting further attempts by the user to set the empty device.

The assembly for adjusting the drug delivery device is applicable, but not limited, to disposable fixed dose injectors. The assembly could be used in variable dose drug delivery devices also.

Other implementations are within the scope of the claims. Elements of different embodiments may be combined to form implementations not specifically described herein. The scope of protection of the invention is not limited to the examples given hereinabove. The invention is embodied in each novel characteristic and each combination of characteristics, which particularly includes every combination of any features which are stated in the claims, even if this feature or this combination of features is not explicitly stated in the claims or in the examples.

### Reference numerals

- 1: body element
- 2: distal housing
- 3: proximal housing
- 4: cartridge
- 5: lead screw / piston rod
- 6: bung
- 7: second thread
- 8: third thread
- 9: nut member
- 10: first thread
- 11: inner drive sleeve
- 12: outer drive sleeve
- 13: fourth thread
- 14: bearing
- 15: stop face
- 16: barrel
- 17: barrel splines
- 18: first arresting means
- 19: second arresting means
- 20: button
- 21: detent means
- 22: button splines
- 24: clutch tooth
- 25: proximal part of button
- 26: clutch tooth
- 27: body insert
- 28: gap
- 29: ramp
- 30: proximal end of inner drive sleeve
- 31: distal spring means
- 32: cut-out
- 33: proximal spring means
- 34: helical structure
- 35: last dose stop
- 50: proximal direction
- 51: distal direction

## Claims

1. An assembly for a drug delivery device comprising
a piston rod (5),
a first drive member (11) for driving the piston rod (5),
a second drive member (12), **characterised in that**
in a first state of the assembly the first drive member (11) is moveable with respect to a second drive member (12) and the piston rod (5) is configured to be moved with respect to the first drive member (11) when the first drive member (11) is moved with respect to the second drive member (12), and
a coupling member (20) being configured to couple the first drive member (11) with the second drive member (12) in a second state of the assembly, so that the movement of the first drive member (11) with respect to the second drive member (12) is prevented, thereby forming a drive unit (11, 12) for a dose setting and delivery action of the drug delivery device, wherein the first drive member (11) and the second drive member (12) do not move relative to each other during the dose setting and delivery action.

2. The assembly according to claim 1, wherein in the first state of the assembly the first drive member (11) is rotationally moveable with respect to the second drive member (12).

3. The assembly according to claim 1 or 2, wherein the first drive member (11) and the piston rod (5) are threadedly engaged (8, 10).

4. The assembly according to any of the claims 1 to 3, wherein the first drive member (11) is at least partly positioned within the second drive member (12).

5. The assembly according to any of the claims 1 to 4, wherein in the first state of the assembly the coupling element (20) is moveable with respect to the second drive member (12), thereby moving the first drive member (11) with respect to the second drive member (12).

6. The assembly according to claim 5, wherein the coupling member (20) comprises a first clutch means (26) and wherein the first drive member (11) comprises a second clutch means (24) configured to be coupled with the first clutch means (26) so that the first drive member (11) rotates with respect to the second drive member (12) when the coupling member (20) rotates with respect to the second drive member (12).

7. The assembly according to any of the claims 1 to 6, wherein one of the second drive member (12) and the coupling member (20) is configured to engage with the other one of the second drive member (12) and the coupling member (20) in the first state so that the coupling member (20) is rotationally moveable with respect to the second drive member (12) in the first state of the assembly, and wherein one of the second drive member (12) and the coupling member (20) is configured to engage with the other one of the second drive member (12) and the coupling member (20) in the second state so that the coupling member (20) is rotationally fixed with respect to the second drive member (12) in the second state of the assembly.

8. The assembly according to claim 7, wherein one of the second drive member (12) and the coupling member (20) comprises a first engagement means (17) configured to engage with a second engagement means (22) which is comprised by the other one of the second drive member (12) and the coupling member (20) so that the movement of the first drive member (11) with respect to the second drive member (12) is prevented in the second state of the assembly.

9. The assembly according to claim 7 or 8, wherein the state of the assembly changes from the first state to the second state when the coupling member (20) moves with respect to the second drive member (12).

10. A body element (1) of a drug delivery device being configured to be coupled with a cartridge (4) which comprises a bung (6), the body element (1) comprising the assembly according to any of the previous claims, the assembly being positioned at least partly inside a housing (3) of the body element (1), wherein the piston rod (5) is configured to move towards the bung (4) when the first drive member (11) is moved with respect to the second drive member (12).

11. The body element (1) according to claim 10, wherein the piston rod (5) is configured to move towards the bung (6) and to abut the bung (6).

12. The body element (1) according to claim 10 or 11, wherein the housing (3) comprises a stop member (15) configured to stop a distal movement of the second drive (12) member with respect to the housing (3) in the first state of the assembly.

13. The body element (1) according to any of the claims 10 to 12, wherein the drive unit (11, 12) is suitable for moving the piston rod (5) in the distal direction with respect to the housing (3), thereby moving the bung (6) in the distal direction with respect to the cartridge (4).

14. The body element (1) according to any of the claims 10 to 13, wherein the coupling element (20) serves as a button (20) for initiating the set and delivery action of a drive mechanism which comprises the piston rod (5).

15. A method for assembling a drug delivery device comprising a body element (1) having a drive mechanism, the body element (1) being coupled with a cartridge (4) having a bung (6), the method comprising:
moving a first drive member (11) with respect to a second drive member (12), thereby moving a piston rod (5) towards the bung (6) during an assembly action, and
coupling the first drive member (11) with the second drive member (12) so that the movement of the first drive member (11) with respect to the second drive member (12) is prevented during a dose setting and delivery action.

## Patentansprüche

1. Anordnung für eine Arzneimittelabgabevorrichtung, umfassend
eine Kolbenstange (5),
ein erstes Antriebselement (11) zum Antreiben der Kolbenstange (5) und
ein zweites Antriebselement (12), **dadurch gekennzeichnet, dass**
das erste Antriebselement (11) in einem ersten Zustand der Anordnung bezüglich eines zweiten Antriebselements (12) beweglich ist und die Kolbenstange (5) so konfiguriert ist, dass sie bezüglich des ersten Antriebselements (11) bewegt wird, wenn das erste Antriebselement (11) bezüglich des zweiten Antriebselements (12) bewegt wird, und
ein Koppelelement (20) so konfiguriert ist, dass es in einem zweiten Zustand der Anordnung das erste Antriebselement (11) an das zweite Antriebselement (12) ankoppelt, so dass die Bewegung des ersten Antriebselements (11) bezüglich des zweiten Antriebselements (12) verhindert wird, wodurch eine Antriebseinheit (11, 12) für einen Dosiseinstell- und' Abgabevorgang der Arzneimittelabgabevorrichtung gebildet wird, wobei sich das erste Antriebselement (11) und das zweite Antriebselement (12) während des Dosiseinstell- und Abgabevorgangs nicht bezüglich zueinander bewegen.

2. Anordnung nach Anspruch 1, wobei das erste Antriebselement (11) im ersten Zustand der Anordnung bezüglich des zweiten Antriebselements (12) drehbeweglich ist.

3. Anordnung nach Anspruch 1 oder 2, wobei das erste Antriebselement (11) und die Kolbenstange (5) in Gewindeeingriff (8, 10) stehen.

4. Anordnung nach einem der Ansprüche 1 bis 3, wobei das erste Antriebselement (11) mindestens teilweise im zweiten Antriebselement (12) positioniert ist.

5. Anordnung nach einem der Ansprüche 1 bis 4, wobei das Koppelelement (20) im ersten Zustand der Anordnung bezüglich des zweiten Antriebselements (12) beweglich ist, wodurch das erste Antriebselement (11) bezüglich des zweiten Antriebselements (12) bewegt wird.

6. Anordnung nach Anspruch 5, wobei das Koppelelement (20) ein erstes Kupplungsmittel (26) umfasst und wobei das erste Antriebselement (11) ein zweites Kupplungsmittel (24) umfasst, das so konfiguriert ist, dass es an das erste Kupplungsmittel (26) gekoppelt wird, so dass sich das erste Antriebselement (11) bezüglich des zweiten Antriebselements (12) dreht, wenn sich das Koppelelement (20) bezüglich des zweiten Antriebselements (12) dreht.

7. Anordnung nach einem der Ansprüche 1 bis 6, wobei das zweite Antriebselement (12) oder das Koppelelement (20) so konfiguriert ist, dass es im ersten Zustand in das jeweils andere des zweiten Antriebselements (12) und des Koppelelements (20) eingreift, so dass das Koppelelement (20) im ersten Zustand der Anordnung bezüglich des zweiten Antriebselements (12) drehbeweglich ist, und wobei das zweite Antriebselement (12) oder das Koppelelement (20) so konfiguriert ist, dass es im zweiten Zustand in das jeweils andere des zweiten Antriebselements (12) und des Koppelelements (20) eingreift, so dass das Koppelelement (20) im zweiten Zustand der Anordnung bezüglich des zweiten Antriebselements (12) drehfest ist.

8. Anordnung nach Anspruch 7, wobei das zweite Antriebselement (12) oder das Koppelelement (20) ein erstes Eingriffsmittel (17) umfasst, das so konfiguriert ist, dass es in ein von dem jeweils anderen des zweiten Antriebselements (12) und des Koppelelements (20) umfasstes zweites Eingriffsmittel (22) eingreift, so dass die Bewegung des ersten Antriebselements (11) bezüglich des zweiten Antriebselements (12) im zweiten Zustand der Anordnung verhindert wird.

9. Anordnung nach Anspruch 7 oder 8, wobei der Zustand der Anordnung vom ersten Zustand zum zweiten Zustand wechselt, wenn sich das Koppelelement (20) bezüglich des zweiten Antriebselements (12) bewegt.

10. Körperelement (1) einer Arzneimittelabgabevorrichtung, das so konfiguriert ist, dass es an eine Patrone (4) gekoppelt wird, die einen Stopfen (6) umfasst, wobei das Körperelement (1) die Anordnung nach einem der vorhergehenden Ansprüche umfasst, wobei die Anordnung mindestens teilweise in einem Gehäuse (3) des Körperelements (1) positioniert ist, wobei die Kolbenstange (5) so konfiguriert ist, dass sie sich zum Stopfen (6) hin bewegt, wenn das erste Antriebselement (11) bezüglich des zweiten Antriebselements (12) bewegt wird.

11. Körperelement (1) nach Anspruch 10, wobei die Kolbenstange (5) so konfiguriert ist, dass sie sich zum Stopfen (6) hin bewegt und an den Stopfen (6) anschlägt.

12. Körperelement (1) nach Anspruch 10 oder 11, wobei das Gehäuse (3) ein Anschlagelement (15) umfasst, das so konfiguriert ist, dass es eine distale Bewegung des zweiten Antriebselements (12) bezüglich des Gehäuses (3) im ersten zustand der Anordnung stoppt.

13. Körperelement (1) nach einem der Ansprüche 10 bis 12, wobei die Antriebseinheit (11, 12) geeignet ist, die Kolbenstange (5) in die distale Richtung bezüglich des Gehäuses (3) zu bewegen, wodurch der Stopfen (6) bezüglich der Patrone (4) in die distale Richtung bewegt wird.

14. Körperelement (1) nach einem der Ansprüche 10 bis 13, wobei das Koppelelement (20) als eine Taste (20) zur Einleitung des Einstell- und Abgabevorgangs eines Antriebsmechanismus, der die Kolbenstange (5) umfasst, dient.

15. Verfahren zur Montage einer Arzneimittelabgabevorrichtung, die ein Körperelement (1) mit einem Antriebsmechanismus umfasst, wobei das Körperelement (1) an eine Patrone (4) mit einem Stopfen (6) gekoppelt ist, wobei das Verfahren folgende Schritte umfasst:
Bewegen eines ersten Antriebselements (11) bezüglich eines zweiten Antriebselements (12), wodurch eine Kolbenstange (5) während eines Montagevorgangs zum Stopfen (6) hin bewegt wird, und
Koppeln des ersten Antriebselements (11) an das zweite Antriebselement (12), so dass die Bewegung des ersten Antriebselements (11) bezüglich des zweiten Antriebselements (12) während eines Dosiseinstell- und Abgabevorgangs verhindert wird.

## Revendications

1. Assemblage pour un dispositif distributeur de médicaments comprenant
une tige de piston (5),
un premier élément d'entraînement (11) pour entraîner la tige de piston (5),
un second élément d'entraînement (12), **caractérisé en ce que**
dans un premier état de l'assemblage, le premier élément d'entraînement (11) est mobile par rapport à un second élément d'entraînement (12) et la tige de piston (5) est configurée pour être déplacée par rapport au premier élément d'entraînement (11) quand le premier élément d'entraînement (11) est déplacé par rapport au second élément d'entraînement (12), et
un élément de couplage (20) étant configuré pour coupler le premier élément d'entraînement (11) avec le second élément d'entraînement (12) dans un second état du assemblage, de sorte que le mouvement du premier élément d'entraînement (11) par rapport au second élément d'entraînement (12) soit évité, formant ainsi une unité d'entraînement (11, 12) pour une action de réglage de dose et de distribution du dispositif de distribution de médicament, dans lequel le premier élément d'entraînement (11) et le second élément d'entraînement (12) ne se déplacent pas l'un par rapport à l'autre au cours de l'action de réglage de dose et de distribution.

2. Assemblage selon la revendication 1, où dans le premier état du assemblage, le premier élément d'entraînement (11) est mobile de manière rotationnelle par rapport au second élément d'entraînement (12).

3. Assemblage selon la revendication 1 ou 2, où le premier élément d'entraînement (11) et la tige de piston (5) sont enclenchés de manière filetée (8, 10).

4. Assemblage selon l'une quelconque des revendications 1 à 3, où le premier élément d'entraînement (11) est au moins partiellement positionné dans le second élément d'entraînement (12).

5. Assemblage selon l'une quelconque des revendications 1 à 4, où dans le premier état de l'assemblage, l'élément de couplage (20) est mobile par rapport au second élément d'entraînement (12) déplaçant ainsi le premier élément d'entraînement (11) par rapport au second élément d'entraînement

6. Assemblage selon la revendication 5, où l'élément de couplage (20) comprend un premier moyen d'embrayage (26) et où le premier élément d'entraînement (11) comprend un second moyen d'embrayage (24) configuré pour être couplé avec le premier moyen d'embrayage (26) de sorte que le premier élément d'entraînement (11) pivote par rapport au second élément d'entraînement (12) quand l'élément de couplage (20) pivote par rapport au second élément d'entraînement (12).

7. Assemblage selon l'une quelconque des revendications 1 à 6, où un du second élément d'entraînement (12) et l'élément de couplage (20) est configuré pour s'enclencher avec l'autre du second élément d'entraînement (12) et l'élément de couplage (20) dans le premier état, de sorte que l'élément de couplage (20) soit mobile de manière pivotable par rapport au second élément d'entraînement (12) dans le premier état du assemblage, et où un du second élément d'entraînement (12) et l'élément de couplage (20) est configuré pour s'enclencher avec l'autre du second élément d'entraînement (12) et l'élément de couplage (20) dans le second état de sorte que l'élément de couplage (20) soit fixé de manière pivotable par rapport au second élément d'entraînement (12) dans le second état du assemblage.

8. Assemblage selon la revendications 7, où un du second élément d'entraînement (12) et l'élément de couplage (20) comprend un premier moyen d'enclenchement (17) configuré pour s'enclencher avec un second moyen d'enclenchement (22) qui est compris par l'autre du second élément d'entraînement (12) et l'élément de couplage (20) de sorte que le mouvement du premier élément d'entraînement (11) par rapport au second élément d'entraînement (12) soit évité dans le second état du assemblage.

9. Assemblage selon la revendication 7 ou 8, où l'état de l'assemblage change depuis le premier état vers le second état quand l'élément de couplage (20) se déplace par rapport au second élément d'entraînement (12).

10. Élément du corps (1) d'un dispositif de distribution de médicaments étant configuré pour être couplé avec une cartouche (4) qui comprend une bonde (6), l'élément de corps (1) comprenant l'assemblage selon l'une quelconque des revendications précédentes, l'assemblage étant positionné au moins partiellement dans un logement (3) de l'élément du corps (1), où la tige de piston (5) est configurée pour se déplacer vers la bonde (4) quand le premier élément d'entraînement (11) se déplace par rapport au second élément d'entraînement (12).

11. Élément du corps (1) selon la revendication 10, où la tige de piston (5) est configurée pour se déplacer vers la bonde (4) et pour jouxter la bonde (6).

12. Élément du corps (1) selon la revendication 10 ou 11, où le logement (3) comprend un élément d'arrêt (15) configuré pour arrêter un mouvement distal du second élément d'entraînement (12) par rapport au logement (3) dans le premier état de l'assemblage.

13. Élément du corps (1) selon l'une quelconque des revendications 10 à 12, où l'unité d'entraînement (11, 12) est appropriée pour déplacer la tige de piston (5) dans le sens distal par rapport au logement (3), déplaçant ainsi la bonde (6) dans le sens distal par rapport à la cartouche (4).

14. Élément du corps (1) selon l'une quelconque des revendications 10 à 13, où l'élément de couplage (20) sert de bouton (20) pour initier l'action de réglage et de distribution d'un mécanisme à entrainement qui comprend la tige de piston (5).

15. Procédé pour assembler un dispositif de distribution de médicaments comprenant un élément de corps (1), ayant un mécanisme d'entraînement, l'élément de corps (1) étant couplé avec une cartouche (4) ayant une bonde (6), le procédé comprenant :
le déplacement d'un premier élément d'entraînement (11) par rapport à un second élément d'entraînement (12), déplaçant ainsi une tige de piston (5) vers la bonde (6) au cours d'une action de assemblage, et
le couplage du premier élément d'entraînement (11) avec le second élément d'entraînement (12) de sorte que le mouvement du premier élément d'entraînement (11) par rapport au second élément d'entraînement (12) soit évité au cours d'une action de réglage de dose et de distribution.
